# EUROPÄISCHE PATENTSCHRIFT

(11) **EP 0 902 858 B1**
(45) Veröffentlichungstag und Bekanntmachung des Hinweises auf die Patenterteilung: **22.03.2000**
(21) Anmeldenummer: 97925039.6
(22) Anmeldetag: 04.06.1997
(51) Int. Cl.: E21B 25/00

(54) **SÄULENVERSCHLUSSKAPPE UND VERFAHREN ZUR VORBEREITUNG UND DURCHFÜHRUNG VON SÄULENUNTERSUCHUNGEN**
COLUMN CLOSURE CAP AND METHOD OF PREPARING AND CARRYING OUT COLUMN INVESTIGATIONS
BOUCHON DE FERMETURE DE COLONNE ET PROCEDE PERMETTANT DE PREPARER ET D'EFFECTUER DES INSPECTIONS DANS DES COLONNES

(30) Priorität: 04.06.1996 DE 19623780
(43) Veröffentlichungstag der Anmeldung: 24.03.1999
(73) Patentinhaber: UFZ-UMWELTFORSCHUNGSZENTRUM LEIPZIG-HALLE GMBH, 04318 Leipzig (DE)
(72) Erfinder: LAZIK, Detlef, D-06108 Halle (DE); SEIFERT, Eckhard, D-01109 Dresden (DE); SANDER, Michael, D-01465 Liegau-Augustusbad (DE); SCHNEIDER, Thomas, D-01109 Dresden (DE)
(74) Vertreter: Hengelhaupt, Jürgen, Dipl.-Ing.
(86) Internationale Anmeldenummer: EP9702884
(87) Internationale Veröffentlichungsnummer: WO9746789

(56) Entgegenhaltungen:
- DD-A- 218 922
- DE-A- 3 434 685
- US-A- 4 014 393

## Beschreibung

Die Erfindung betrifft eine Säulenverschlußkappe und ein Verfahren zur Vorbereitung und Durchführung von Säulenuntersuchungen unter Verwendung derartiger Kappen und ist anwendbar insbesondere zur Gewinnung ungestörter Boden- und Gesteinsproben zur Vorbereitung und Durchführung von Migrationsuntersuchungen in Umlauf- oder Durchlaufsäulen oder auch für Säulenversuche mit gestört eingebautem Probenmaterial.

Die Planung und Auswahl geeigneter Umweltsanierungsmaßnahmen erfolgt in vielen Anwendungsfällen nach umfangreichen Untersuchungen an Boden- und Wasserproben im Labor. Wichtige Aussagen hierfür liefern dynamische Migrationsversuche in Säulenanlagen, die der Simulation der Transport-, Austausch- und Umwandlungsprozesse im Untergrund dienen. Sie ermöglichen die Analyse der Ursache - Wirkungszusammenhänge, so daß vielfältige Maßnahmen, zum Beispiel der Grundwasseraufbereitung oder der Altlastensanierung, mit relativ geringem experimentellen Aufwand optimal gestaltet werden können.

Es ist bekannt, die Proben in dem zu untersuchenden Gelände im Zuge von Bohrarbeiten mittels Kernen zu entnehmen. Hierbei wird ein im Bohrgestänge laufendes Kunststoff-Rohr (der Liner) mit dem Einpressen des Gestänges in das Erdreich durch einen weitgehend ungestörten Boden- beziehungsweise Gesteinskern gefüllt. Nach dem Ziehen des Bohrgestänges wird der probengefüllte Liner entnommen und steht nun u.a. für Migrationsuntersuchungen in Umlauf- oder Durchlaufsäulen zur Verfügung.

Traditionell wird der probengefüllte Liner durch mittels Wachs oder Klebeband gedichtete Kappen beidseitig verschlossen. Nachfolgend wird der Liner gegebenenfalls konserviert und zur Bearbeitung ins Labor transportiert beziehungsweise in Tiefkühltruhen zwischengelagert. Die Bodenprobe wird dann im tiefgefrorenen Zustand auf Endmaße gesägt, belüftete Bereiche werden entfernt und die auf Maß geschnittenen Stücke in eine bereitgestellte Säule eingepreßt. Die Säule wird verschlossen und in den Versuchskreis eingebaut. Der entleerte Liner wird verworfen.

Nachteilig bei der bekannten Verfahrensweise ist, daß die Proben nach ihrer Lagerung in hierfür extra bereitzustellende Säulen eingepreßt werden müssen. Neben der Tatsache, daß die Säulen relativ teuer sind, wirkt sich insbesondere die mechanische Beanspruchung der Probe beim Einpressen sowie die nochmalige Belüftung negativ auf die Untersuchungsergebnisse aus.

In der DE-U-94 120 12.9 wird eine formschlüssige Krallenringverschraubung zur Druckprüfung von Rohren beschrieben, deren konisch ausgebildeter Krallenring durch das Anziehen eines Spannringes in das zu prüfende Rohr einschneidet. Als separaten Teil enthält der Prüfverschluß eine Vorrichtung zur Dichtung.

Nachteilig an dieser Lösung ist, daß die Dichtung durch eine relativ komplizierte Vorrichtung realisiert wird und eine Materialzerstörung an den Außenflächen des Rohres erfolgt.

Der Erfindung liegt daher die Aufgabe zugrunde, eine Säulenverschlußkappe zur Vorbereitung und Durchführung von Säulenuntersuchungen an Bohrproben aus dem Erdreich zu schaffen, welche einfach und preiswert herstellbar ist, einen beidseitig dichten Abschluß des Liners gewährleistet, unter Feldbedingungen montierbar ist und eine Wiederverwendung gestattet.
Es ist weiterhin Aufgabe der Erfindung, ein Verfahren zur Vorbereitung und Durchführung von Säulenuntersuchungen an Bohrproben unter Verwendung der beschriebenen Säulenverschlußkappen zu schaffen, welches den Wegfall der traditionellen Säulen ermöglicht und mechanische Beanspruchungen sowie zusätzliche Belüftungen vermeidet.

Diese Aufgabe wird erfindungsgemäß gelöst durch die Merkmale im kennzeichnenden Teil der Ansprüche 1 und 12 in Verbindung mit den Merkmalen im jeweiligen Oberbegriff. Zweckmäßige Ausgestaltungen der Erfindung sind in den Unteransprüchen enthalten.

Ein besonderer Vorteil der Erfindung besteht darin, daß die Säulenverschlußkappe universal einsetzbar ist und im an dem Liner montierten Zustand die traditionellen Säulen ersetzt, indem die Liner-Endkappe zweiteilig ausgebildet ist und eine Kappe sowie mindestens einen Spannring aufweist, wobei zwischen Kappe und Spannring ein O-Ring angeordnet ist und eine dichtende Verbindung zu dem Liner gewährleistet. Die Dichtung erfolgt auf dem Linerumfang, der Liner muß nicht plangeschnitten sein, um mit der Säulenverschlußkappe zu dichten.
Für die Durchführung der Versuche wird Realitätsnähe gewährleistet durch weitgehend anaerobe Versuchsbedingungen, Abschirmung der Probenmedien vor Licht und durch die Möglichkeit zur Konstanthaltung der Temperatur der Bodenproben sowie der Flüssigkeit im Vorratsgefäß.

Durch die spezielle konische Gestaltung eines Ein/Auslaufverteilerraumes in der Kappe im Zusammenwirken mit einem Dichtring unter der Filterplatte werden Randumströmungen verhindert und es wird eine Paralleldurchströmung des probengefüllten Liners erzwungen.

Ein weiterer Vorteil der Erfindung besteht darin, daß der Einsatz von mehreren Filterplatten in Sandwichkonstruktion möglich ist und Durchbrüche an Stirnfläche und Umfang eine variable Gestaltung von Zu- und Abfluß, die Ent- und Belüftung, Tracerzugabe und Messungen ermöglichen. Die Durchbrüche sind mit selbstschließenden Schnellspannkupplungen bestückbar, um Flüssigkeitsverluste oder den Zutritt von Luft zu vermeiden. Vorteilig ist auch die Wiederverwendbarkeit der Kappe.

Darüberhinaus ist ein variabler Versuchsaufbau möglich, wobei hydraulisch stationäre und instationäre Strömungsregime realisiert und gesättigte sowie ungesättigte Verhältnisse simuliert werden können. Durch die variable Anordnung der Säulen und Säulenelemente wird ein universelles Baukastensystem gebildet, wobei einzelne Bauteile zur Realisierung anderer Versuchsanlagen multivalent genutzt werden können.

Neben den Veränderungsmöglichkeiten am Versuchsaufbau gibt es auch vorteilhafte Meßmöglichkeiten, beispielsweise die Messung des Kapillardrucks in der Bodenprobe zur Kontrolle des sich einstellenden Strömungsregimes als Basis der Steuerung der Versuchsanlage, die Messung der Leitfähigkeit der Lösung nach jedem Säulenelement und durch Anschluß der Durchflußmeßzelle eine pH-, Redox-, Sauerstoff- und Temperaturmessung nach jedem Säulenelement. Weiterhin ist die Einbindung weiterer Sensoren, zum Beispiel TDRSensorik oder ionenselektiver Sensorik, optional möglich.

Ein zusätzlicher Vorteil der Erfindung resultiert daraus, daß die Spannschrauben in einer verlängerten Ausführung gleichzeitig den Fluß des Säulenaufbaus bilden.

Die Erfindung soll nachstehend anhand von in den Figuren zumindest teilweise dargestellten Ausführungsbeispielen näher erläutert werden. Es zeigen:
- Fig. 1: eine schematische Darstellung durch eine Säulenverschlußkappe mit zugehöriger Draufsicht
- Fig. 2: eine stilisierte Darstellung eines Säulenversuches unter Einsatz der Säulenverschlußkappe

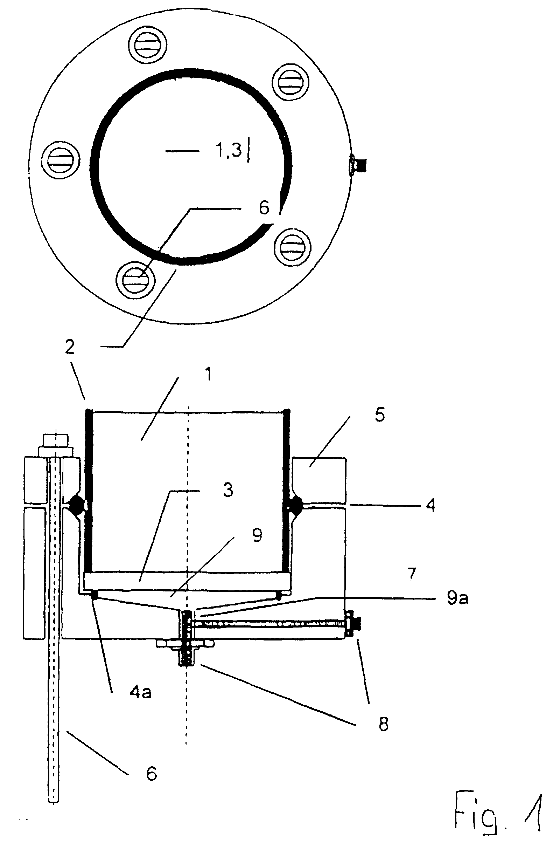

Wie aus Fig. 1 zu ersehen ist, befindet sich in einem Liner 2 ein Gesteinskern 1.
Die zwei Säulenverschlußkappen, bestehend aus Kappe 7 und Spannring 5, wurden bereits im Feld unmittelbar nach Entnahme des probegefüllten Liners 2 beidseitig auf den Liner 2 geschoben und die Spannschrauben 6 angezogen. Hierdurch wird der O-Ring 4 gegen den Liner 2 gepreßt und realisiert eine sichere Befestigung sowie langzeitwirksame Abdichtung.

Zwischen dem Liner 2 und dem Ein-/Auslaufverteilerraum 9 in der Kappe 7 ist im vorliegenden Ausführungsbeispiel eine poröse Platte 3 angeordnet. Der Ein/Auslaufverteilerraum 9 ist konisch ausgebildet und weist an seinem tiefsten Punkt einen Ein- beziehungsweise Auslauf 9a auf. Zwischen der porösen Platte 3 und dem Ein-/Auslaufverteilerraum 9 ist ein Dichtring 4a angeordnet, welcher Randumströmungen verhindert.
Selbstverständlich ist es auch möglich, mehrere poröse Platten im Sandwichaufbau oder zusätzlich Gaze beispielweise mit definiertem Lufteintrittspunkt derart anzuordnen, daß der Lufteintritt in den Auslaufverteiler bei der Untersuchung ungesättigter Bodensäulen vermieden wird.
Im vorliegenden Ausführungsbeispiel ist der Ein-/Auslauf 9a in Richtung des Bohrkerns 1 und senkrecht zum Bohrkern 1 aus der Kappe 7 herausgeführt und mit Schlauchanschlüssen 8 versehen. Die Schlauchanschlüsse 8 sind selbstschließende Schnellkupplungen. Die Spannschrauben 6 bilden gleichzeitig in einer verlängerten Ausführung den Fuß des Säulenaufbaus.

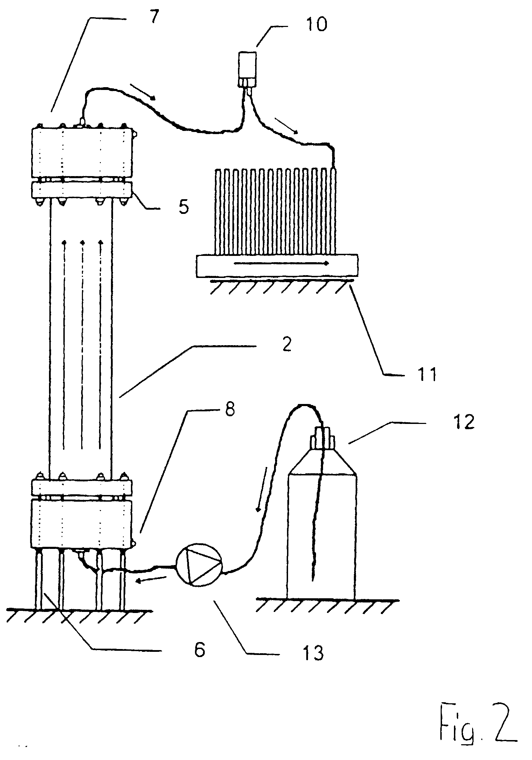

Figur 2 zeigt einen derartigen Säulenversuchsaufbau, bei welchem die Säule durch zwei auf dem Liner 2 aufgebrachte Säulenverschlußkappen gebildet wird und die verlängerten Spannschrauben 6 gleichzeitig den Fuß der Säule bilden.

Nachfolgend soll am Beispiel eines Durchlaufsäulenversuches der Verfahrensablauf kurz beschrieben werden.

Bei einem Durchlaufsäulenversuch unter wassergesättigten Fließverhältnissen wird eine gesteinsgefüllte Säule 2 durch Wasser aus einem Vorratsbehälter 12 laminar durchströmt. Duch die Ausrichtung des Volumenstroms entgegen der Schwerkraft wird die Entgasung des Fluid-Feststoffssystems unterstützt und ein möglicher Partikelaustrag gebremst.
Der zum Heben des Wasses bis zum Potentialgefäß 10 notwendige Wasserdruck kann durch eine Schlauchpumpe 13 erzeugt werden. Das Potentialgefäß 10 dient neben der Festlegung eines definierten Druckgefälles, der Entkopplung des auf der Säule 2 stehenden Wasserdrucks vom Probenahmesystem und der Abführung von Gasbläschen. Zur Probenahme kann beispielsweise ein Fraktionensammler 11 eingesetzt werden, der die automatische Befüllung von Probenahmeflaschen mit dem ausfließenden Wasservolumen während programmierbarer Zeitabstände ermöglicht. Die einzelnen Wasserfraktionen werden nun bezüglich der Konzentrationen interessierter Inhaltsstoffe untersucht.
Ggf. kann ein weiteres Fluid, das einen Tracer oder einen Stoff enthält, dessen Migrationsverhalten studiert werden soll, duch den seitlichen Schlauchanschluß 8 zugesetzt werden.

Anschließend sollen die wesentlichen, vorteilhaften Eigenschaften der Säulenverschlußkappe noch einmal zusammengefaßt aufgeführt werden.
1. Dichtung erfolgt auf dem Linerumfang, Liner muß nicht plan geschnitten sein, um zu dichten.
2. Nullring unter Filterplatte verhindert Randumströmung.
3. Einsatz von mehreren Filterplatten in Sandwichkonstruktion möglich.
4. Kappe enthält Ein- bzw. Auslaufverteiler (konisch unter Filterplatte) zur parallelen Säulenanströmung.
5. Durchbrüche an Stirnfläche und Umpfang ermöglichen eine variable Gestaltung von Zu- und Abfluß, die Entlüftung und Tracereingabe.
6. Durchbrüche sind mit Schnellspannkupplungen (selbstschließend) bestückbar.
7. Kappe ist wiederverwendbar.
8. Traditioneller Säulenaufbau in Flanschkonstruktion wird durch flexibel zusammenstellbares Baukastensystem für Säulenversuche für unterschiedlichste Aufgabenstellungen ersetzt.

Die Erfindung ist nicht beschränkt auf die hier beschriebenen Ausführungsbeispiele. Vielmehr ist es möglich, durch geeignete Kombination der genannten Mittel und Merkmale weitere Ausführungsvarianten zu realisieren, ohne den Rahmen der Erfindung zu verlassen.

### Bezugszeichenliste

- 1: Bohrkern
- 2: Säulenverschlußkappe
- 3: poröse Platte
- 4: O-Ring
- 4a: Dichtring
- 5: Spannring
- 6: Spannschrauben
- 7: Kappe
- 8: Schlauchanschlüsse
- 9: Ein-/Auslaufverteilerraum
- 9a: Ein-/Auslauf
- 10: Potentialgefäß
- 11: Fraktionssammler
- 12: Vorratsbehälter
- 13: Schlauchpumpe

## Patentansprüche

1. Säulenverschlußkappe zur Vorbereitung und Durchführung von Säulenuntersuchungen an Bohrproben aus dem Erdreich, wobei
die Säulenverschlußkappe zweiteilig ausgebildet ist und eine Kappe (7) sowie mindestens einen Spannring (5) aufweist, wobei zwischen Kappe (7) und Spannring (5) ein O-Ring (4) angeordnet ist und eine dichtende Verbindung zur Säule (Liner) (2) gewährleistet und die Kappe (7) einen Ein-/Auslaufverteilerraum (9) mit Ein-/Auslauf (9a) aufweist.

2. Säulenverschlußkappe nach Anspruch 1,
dadurch gekennzeichnet, daß
die Kappe (7) und der Spannring (5) durch Spannschrauben (6) miteinander verbunden sind.

3. Säulenverschlußkappe nach Anspruch 1,
dadurch gekennzeichnet, daß
der Ein-/Auslaufverteilerraum (9) konisch ausgebildet ist.

4. Säulenverschlußkappe nach Anspruch 1,
dadurch gekennzeichnet, daß
zwischen dem Liner (2) mit Bohrkern (1) und dem Ein-/Auslaufverteilerraum (9) mindestens eine poröse Platte (3) angeordnet ist.

5. Säulenverschlußkappe nach Anspruch 4,
dadurch gekennzeichnet, daß
unter der porösen Platte (3) und/ oder zwischen Platte (3) und Bohrkern (1) zusätzlich eine Gaze angeordnet ist.

6. Säulenverschlußkappe nach Anspruch 1,
dadurch gekennzeichnet, daß
der Ein-/Auslauf (9a) in Richtung des Bohrkerns (1) und/oder senkrecht zum Bohrkern (1) aus der Kappe (7) herausgeführt und mit Schlauchanschlüssen (8) versehen ist.

7. Säulenverschlußkappe nach Anspruch 6,
dadurch gekennzeichnet, daß
die Schlauchanschlüsse (8) selbstschließende Schnellkupplungen sind.

8. Säulenverschlußkappe nach mindestens einem der voranstehenden Ansprüche,
dadurch gekennzeichnet, daß
zwischen der porösen Platte (3) und dem Boden des Ein-/Auslaufverteilers (9) ein Dichtring (4a) angeordnet ist.

9. Säulenverschlußkappe nach mindestens einem der voranstehenden Ansprüche,
dadurch gekennzeichnet, daß
die Spannschrauben (6) in einer verlängerten Ausführung gleichzeitig den Fuß des Säulenaufbaus bilden.

10. Säulenverschlußkappe nach mindestens einem der voranstehenden Ansprüche,
dadurch gekennzeichnet, daß
die Säulenverschlußkappe wiederverwendbar ist.

11. Verfahren zur Vorbereitung und Durchführung von Säulenuntersuchungen an Bohrproben aus dem Erdreich, wobei ein vorzugsweise als Kunststoffrohr ausgebildeter Liner im Bohrgestänge mit der Bohrprobe gefüllt, anschließend entnommen und die Bodenprobe Untersuchungen zugeführt wird,
dadurch gekennzeichnet, daß
- der Liner unmittelbar nach der Entnahme im Feld endgültig beidseitig mit einer spezifisch ausgebildeten zweiteiligen, wiederverwendbaren Säulenverschlußkappe verschlossen wird,
- der Liner mit den beiden Säulenverschlußkappen selbst die Säule bildet und in den Versuchskreis eingebaut wird und
- der Liner mit Probe nach dem Ende des Säulenversuches entsprechend der Aufgabenstellung weiterverwendet wird und die Endkappen gereinigt und wiederverwendet werden.

12. Verfahren nach Anspruch 11,
dadurch gekennzeichnet, daß
der Liner mit Endkappen entsprechend seiner Entnahmetemperatur temperiert transportiert und/oder aufbewahrt wird.

13. Verfahren nach Anspruch 11,
dadurch gekennzeichnet, daß
der Liner vor endgültigem Verschluß mit den beiden Säulenverschlußkappen auf die Säulenendmaße gekürzt werden kann.

## Claims

1. A column closure cap for preparing and performing column investigations on core samples from the soil, wherein
the column closure cap is of a two-part design and has a cap (7) and at least one tension ring (5), wherein an O-ring (4) is arranged between the cap (7) and the tension ring (5) ensuring a sealing connection to the column (liner) (2), and the cap (7) has an inlet/outlet distributor space (9) including inlet/outlet (9a).

2. The column closure cap according to claim 1, characterized in that the cap (7) and the tension ring (5) are joined to each other by means of tension screws (6).

3. The column closure cap according to claim 1, characterized in that the inlet/outlet distributor space (9) is of a conical design.

4. The column closure cap according to claim 1, characterized in that at least one porous plate (3) is arranged between the liner (2) including the core sample (1) and the inlet/outlet distributor (9).

5. The column closure cap according to claim 4, characterized in that a gauze is additionally arranged beneath the porous plate (3) and/or between the plate (3) and the core sample (1).

6. The column closure cap according to claim 1, characterized in that the inlet/outlet (9a) leads out of the cap (7) in direction of the core sample (1) and/or vertically to the core sample (1) and is provided with flexible tube connections (8).

7. The column closure cap according to claim 6, characterized in that the flexible tube connections (8) are self-locking quick-action couplings.

8. The column closure cap according to at least one of the preceding claims, characterized in that a sealing ring (4a) is arranged between the porous plate (3) and the bottom of the inlet/outlet distributor (9).

9. The column closure cap according to at least one of the preceding claims, characterized in that the tension screws (6) in an extended embodiment simultaneously constitute the stand of the column construction.

10. The column closure cap according to at least one of the preceding claims, characterized in that the column closure cap is reusable.

11. A method of preparing and performing column investigations on core samples from the soil, wherein a liner in the drill pipe, preferably designed as a plastic tube, is filled with the core sample, subsequently removed, and the core sample is subjected to investigations, characterized in that
- the liner, immediately after removal in the field, is finally sealed on both sides with a specifically designed two-part, reusable column closure cap,
- the liner itself, with its two column closure caps, is forming the column and is integrated in the experimental cycle,
- the liner with the sample, after completion of the column experiment, is put to further use according to the problem definition, and the terminal caps are cleaned and used again.

12. The process according to claim 11, characterized in that the liner with its terminal caps is shipped and/or stored in a tempered state according to its temperature of removal.

13. The process according to claim 11, characterized in that the liner is shortened to the final column dimensions prior to final sealing with both column closure caps.

## Revendications

1. Bouchon de fermeture de colonne afin de préparer et d'effectuer des essais en colonne sur des échantillons de forage provenant du sol, dans lequel le bouchon de fermeture de colonne est réalisé en deux pièces et présente un bouchon (7) ainsi qu'au moins une bague de serrage (5) où entre le bouchon (7) et la bague de serrage (5) est disposé un joint torique (4) et assure une liaison étanche par rapport à la colonne (liner) (2) et le bouchon (7) présente un espace de distribution d'entrée et de sortie (9) avec une entrée et une sortie (9a).

2. Bouchon de fermeture de colonne selon la revendication 1, caractérisé en ce que le bouchon (7) et la bague de serrage (5) sont assemblés l'un à l'autre par des vis de serrage (6).

3. Bouchon de fermeture de colonne selon la revendication 1, caractérisé en ce que l'espace de distribution d'entrée et de sortie (9) présente une forme conique.

4. Bouchon de fermeture de colonne selon la revendication 1, caractérisé en ce que, entre le liner (2) avec la carotte de forage (1) et l'espace de distribution d'entrée et de sortie (9) est disposé au moins une plaque poreuse (3).

5. Bouchon de fermeture de colonne selon la revendication 4, caractérisé en ce que, sous la plaque poreuse (3) et/ou entre la plaque (3) et la carotte de forage (1), est disposé en outre une gaze.

6. Bouchon de fermeture de colonne selon la revendication 1, caractérisé en ce que l'entrée/sortie (9a) est disposée dans la direction de la carotte de forage (1) et/ou perpendiculairement à la carotte de forage (9) en sortant du bouchon (7) et est pourvue de raccords à flexible (8).

7. Bouchon de fermeture de colonne selon la revendication 6, caractérisé en ce que les raccords à flexible (8) sont des accouplements rapides autofermants.

8. Bouchon de fermeture de colonne selon au moins l'une des revendications précédentes, caractérisé en ce qu'entre la plaque poreuse (3) et le fond du distributeur d'entrée et de sortie (9), est disposée une bague d'étanchéité (4a).

9. Bouchon de fermeture de colonne selon au moins l'une des revendications précédentes, caractérisé en ce que les vis de serrage (6) dans un mode de réalisation allongé, forment simultanément le pied du dispositif à colonne.

10. Bouchon de fermeture selon au moins l'une des revendications précédentes, caractérisé en ce que le bouchon de fermeture de colonne est réutilisable.

11. Procédé pour préparer et effectuer des essais en colonne sur des échantillons de forage provenant du sol où un liner, formé de préférence d'un tube de matière plastique, est rempli dans la tige de forage par l'échantillon de forage, est prélevé ensuite et l'échantillon de sol est soumis à des essais, caractérisé en ce que
- le liner est fermé définitivement des deux côtés, immédiatement après le prélèvement sur le terrain au moyen d'un bouchon de fermeture de colonne réutilisable et réalisé spécifiquement en deux pièces,
- le liner forme lui-même la colonne avec les deux bouchons de fermeture de colonne et est incorporé dans le circuit d'essai,
- le liner est réutilisé avec l'échantillon à la fin de l'essai en colonne selon l'objectif envisagé et les bouchons d'extrémité sont nettoyés et réutilisés.

12. Procédé selon la revendication 11, caractérisé en ce que le liner avec les bouchons d'extrémité est transporté, maintenu à la température de prélèvement et/ou est conservé de cette manière.

13. Procédé selon la revendication 11, caractérisé en ce que le liner peut être raccourci avant la fermeture finale avec les deux bouchons de fermeture de colonne à la dimension finale de la colonne.
